(19) Europäisches Patentamt
European Patent Office
Office européen des brevets

(11) Veröffentlichungsnummer : **0 298 020 B1**

# (12) EUROPÄISCHE PATENTSCHRIFT

(45) Veröffentlichungstag der Patentschrift :
**24.06.92 Patentblatt 92/26**

(51) Int. Cl.$^5$ : **C07D 301/12,** C07J 71/00

(21) Anmeldenummer : **88730149.7**

(22) Anmeldetag : **01.07.88**

## (54) Verwendung eines Epoxidierungs-Reagenz, sowie Verfahren zur Herstellung von 5 Alpha(10 Alpha)-epoxi Delta 9,11-steroiden.

(30) Priorität : **03.07.87 DE 3722486**

(43) Veröffentlichungstag der Anmeldung :
**04.01.89 Patentblatt 89/01**

(45) Bekanntmachung des Hinweises auf die Patenterteilung :
**24.06.92 Patentblatt 92/26**

(84) Benannte Vertragsstaaten :
**AT BE CH DE ES FR GB GR IT LI LU NL SE**

(56) Entgegenhaltungen :
**EP-A- 0 005 100**
**DE-A- 2 239 681**
**US-A- 4 344 887**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Nickisch, Klaus, Dr.**
**Alt Lichtenrade 11**
**W-1000 Berlin 49 (DE)**
Erfinder : **Arnold, Hanfried, Dr.**
**Von-Wettstein-Strasse 4**
**W-1000 Berlin 33 (DE)**
Erfinder : **Rohde, Ralph, Dr.**
**Schwatlowstrasse 4**
**W-1000 Berlin 45 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft die Verwendung eines Epoxidierungs-Reagenz in einemVerfahren zur Epoxidierung von organischen Verbindungen.

Das zur Herstellung von epoxidierten Steroiden vorgeschlagene Epoxidierungs-Reagenz besteht aus Wasserstoffperoxid und mindestens einem Arylmethylketon der allgemeinen Formel I

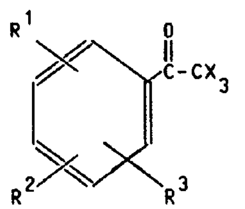

(I),

worin

X für ein Fluor- oder Chloratom,

$R^1$, $R^2$ und $R^3$, die gleich oder verschieden sein können, jeweils für einen $C_1$-$C_6$-Alkyl-, $C_6$-$C_{10}$-Aryl, $C_6$-$C_{10}$-Aryl-$C_1$-$C_6$-alkyl-, 0-$C_1$-$C_6$-Alkyl-, 0-$C_6$-$C_{10}$-Aryl-Rest, ein Fluor-, Chlor-, Brom- oder Wasserstoffatom, eine Cyano-, Nitro- oder Trifluormethylgruppe, einen Amidrest -CO-$NR^4R^5$ oder einen Sulfonylrest -$SO_2R^4$,

worin $R^4$ und $R^5$ gleich oder verschieden sind und jeweils eine $C_1$-$C_6$-Alkyl-, $C_1$-$C_{10}$-Aryl- oder eine $C_6$-$C_{10}$-Aryl-$C_1$-$C_6$-alkylgruppe, wobei im Falle von -CO-$NR^4R^5$

$R^4$ und $R^5$ gemeinsam einen aliphatischen oder aromatischen fünf- oder sechsgliedrigen Heterocyclus bilden können, bedeuten oder $R^1$ und $R^2$ gemeinsam für einen anellierten aliphatischen oder aromatischen Ring stehen.

Als Alkylrest seien Methyl-, Ethyl-, Propyl-, Butyl-, Isopropyl-, Pentyl-, Hexyl genannt, wobei die $C_1$-$C_4$-Reste bevorzugt sind.

Als Arylrest seien Phenyl- und Naphthyl- genannt. Als Aralkylrest ist Benzylbevorzugt.

Stehen $R^1$ und $R^2$ gemeinsam für einen anellierten Ring, so sind der Cyclohexan- und der Phenylring bevorzugt.

Bei der Substitution des Arylketons am Arylrest sind die Mono- und Disubstitution bevorzugt.

Es ist bereits eine Vielzahl von Epoxidierungsreagenzien bekannt. Die gebräuchlichsten dürften basisches Wasserstoffperoxid [s. z.B. J. Org.Chem. 24, 2048 (1959)] sowie die organischen Persäuren [s. z.B. Tetrahedron 39, 2323 (1983)], wie zum Beispiel Perbenzoesäure, Perphthalsäure oder m-Chlorperbenzoesäure, sein. Gegenüber diesen Reagenzien hat das vorgeschlagene Epoxidierungs-Reagenz verschiedene Vorteile; so gestattet es beispielsweise den Einsatz säureempfindlicher sowie labile Gruppen enthaltender Edukte, und es weist eine größere Stereoselektivität auf.

Die in letzter Zeit bekannt gewordenen Reagenzien weisen zwar zum Teil ebenfalls die genannten Vorteile auf, besitzen aber dafür andere Nachteile: die in Tetrahedron 39, 1635 (1983) am Beispiel eines speziellen Olefins (Chalcon, ein $\alpha$, $\beta$-ungesättigtes aromatisches Keton) vorgestellte Methode arbeitet mit nicht käuflichen bzw. teuren Poly-L-Aminosäuren und basischem Wasserstoffperoxid(s.o.) in einem sicherlich nicht für alle Olefine geeigneten wäßrigen Dreiphasensystem. Die von K.B. Sharpless [J. Org. Chem. 47,1378 (1982), J. Org. Chem. 49, 728 (1984)] beschriebenen Titanverbindungen sind nur bei allylischen Alkoholen anwendbar.

Andere Reagenzien sind nicht lange lagerungsfähig [z. B. 3,5-Dinitroperbenzoesäure, J. Org. Chem. 43, 1689 (1978)], sehr teuer [z.B. Hexafluoraceton, Synthesis 810 (1980) und EP 5.100 (1979)] oder schwer zugänglich bzw. sehr teuer [z.B. Porphyrine, J. Am. Chem. Soc. 105, 5786 (1983), Tetrahedron Letters 1984, 789] und ergeben bei ihrem Einsatz nicht immer zufriedenstellende Stereoselektivität.

Das erfindungsgemäss zu verwendende Trifluoracetophenon hat sich lant DE-A-2 239 681 (Tabelle II) als Epoxidierungskatalysator für Octen-1 als unwirksam erwiesen, ebenso - lant US-A-4 344 887 (Table 1) - Dichloracetophenon als Epoxidierungskatalysator für Cyclohexen.

Es bestand also trotz der Vielzahl vorbekannter Epoxidierungs-Reagenzien ein Bedürfnis nach einem neuen, möglichst viele der genannten Nachteile nicht aufweisenden Mittel zur Epoxidierung von Steroiden.

Es wurde nun gefunden, daß mit einem Reagenz, bestehend aus Wasserstoffperoxid und mindestens einem Arylmethylketon der allgemeinen Formel I überraschenderweise diese Aufgabe gelöst wird.

Dabei stehen das Wasserstoffperoxid und das Arylmethylketon der allgemeinen Formel I vorzugsweise in einem Gewichtsverhältnis, bei Wasserstoffperoxid berechnet auf eine 30%ige wäßrige Lsöung, von 1:2 bis 1:20.

Das erfindungsgemäss verwendete Epoxidierungs-Reagenz zeichnet sich dadurch aus, daß es stabil, leicht zugänglich bzw. billig ist und Reaktionen mit hohen Ausbeuten und großer Selektivität ermöglicht. Insbesonders verlaufen die Reaktionen dann sehr schnell und unter milden thermischen Bedingungen (-20°C - Raumtemperatur), wenn der Arylteil des Epoxidierungsreagenzes durch einen oder mehrere elektronenziehende Reste wie Nitro, Cyano, Trifluormethyl oder durch eine Amidgruppe oder eine Sulfonylgruppe substituiert ist; bevorzugt ist dabei die Nitrosubstitution.

Die verwendeten Reagenzien der allgemeinen Formel I sind käuflich erhältlich bzw. nach literaturbekannten Verfahren einfach herstellbar.

So kann man beispielsweise die gewünschte Acetylgruppe durch Friedel-Crafts-Acylierung [J. Org. Chem. 44,313(1979)] in den aromatischen Ring einführen, oder die Acetylgruppe des Aromaten halogenieren (C.A. 84, 30593x; Rocz. Chem. 1973, 47,2333) oder, gewünschtenfalls substituierte, Brom- oder Jodphenyl-Ausgangsprodukte, nach Überführung in die entsprechenden Grignard- oder Lithiumphenyl-Verbindungen mit zum Beispiel Trichlor- oder Trifluoressigsäureanhydrid (DDR-Patentschrift 207715, Can.J.Chem. 58,2491, 1980) oder Trifluormethylformamiddimethoxyacetal (Synth. Comm. 15,1291, 1985) umsetzen.

Auch die Umwandlung von geeignet-substituierten, den gewünschten Acetylsubstituenten bereits enthaltenden Aromaten ist möglich. Als Beispiel sei die Synthese von Chlor- oder Brom-substituierten Trifluoracetophenonen aus den entsprechenden Amino-substituierten Trifluoracetophenonen genannt (J. Org. Chem. 35, 1711, 1970).

Die Ausbeuten, die mit dem vorgeschlagenen Epoxidierungs-Reagenz erreicht werden, sind denen aus dem Stand der Technik zumindest gleichwertig, oft sind sie höher.

Das vorgeschlagene Epoxidierungs-Reagenz bietet den weiteren Vorteil, im Falle von Edukten mit mehr als einer Doppelbindung im Molekül regioselektiv die höher substituierte Doppelbindung anzugreifen. So erhält man beim Umsatz von 5(10),9(11)-Estradien ausschließlich und in guten Ausbeuten die 5(10)Epoxide, während man mit zum Beispiel m-Chlorperbenzoesäure Gemische der 5(10) und 9(11) Epoxide bekommt (Bull. Soc. Chim. 70, 2548).

Zusätzlich zu dieser Regioselektivität erreicht man mit dem vorgeschlagenen Epoxidierungs-Reagenz eine hohe Stereospezifität in bezug auf die Konfiguration des 5(10)-Epoxids: Während man mit den bisher verwendeten Reagenzien ein 5α(10α)/5β(10β)-Epoxid-Isomerenverhältnis von 1:1 bis ca. 2:1 [mit Wasserstoffperoxid/Hexachloraceton: EP 5.100 (1979)] erzielt, entsteht mit dem vorgeschlagenen Epoxidierungs-Reagenz das als Zwischenprodukt für die Herstellung der antigestagen wirkenden 11β-Aryl-4,9(10)-estradien-3-one [Tetrahedron Letters 1979, 2051; Steroids 37, 361 (1981), EP 57.115 (1981)] ausschließlich benötigte 5α, 10α-Epoxid in großem Überschuß. Die Abtrennung des unerwünschten, in geringer Menge mitentstandenen 5β,10β-Epoxids wird dadurch sehr erleichtert.

Im Vergleich zu dem in DE 3 438 484 beschriebenen Epoxidierungs-Reagenz (Übergangsmetall-Phthalocyanin/Jodosobenzol)sind die Ausbeuten des erfindungsgemäßen Reagenzes zum Teil beträchtlich höher (z.B. 86,6% gegenüber 40%; 71% gegenüber 35%, s. experimenteller Teil, Beispiele 2 und 3). Außerdem wird die Verwendung des in DE 3 438 484 bevorzugten Lösungsmittels Acetonitril vermieden, das vor allem bei größeren Ansätzen Probleme wegen seiner toxischen Eigenschaften sowie seines nicht immer ausreichenden Lösungsvermögens verursacht.

Die Erfindung betrifft außerdem ein Verfahren zur Herstellung von Steroiden mit einer 9(11)-Doppelbindung und einer 5α(10α)-Epoxigruppe. Dabei wird eine organische Verbindung der Einwirkung eines Epoxidierungs-Reagenzes bestehend aus Wasserstoffperoxid und mindestens einem Arylmethylketon der allgemeinen Formel I in einem organischen Lösungsmittel unter Zusatz eines tertiären Amins unterworfen und so ein epoxidiertes Produkt erhalten. Man bekommt in hoher Stereo- und Regioselektivität 5α(10α)-Epoxide.

Die Reaktion wird in inerten organischen Lösungsmitteln wie Dioxan, Diglyme, Tetrahydrofuran, Dimethoxyethan, oder in chlorierten organischen Lösungsmitteln unter Zusatz eines tertiären Amins, vorzugsweise in Dichlormethan, Chloroform oder Ethylenchlorid unter Zusatz von Pyridin oder Lutidin durchgeführt.

Als tertiäres Amin kann zum Beispiel Triäthylamin, Trimethylamin,Pyridin, N,N-Dimethylaminopyridin, 1,5-Diazabicyclo[4.3.0]nonen-5 (DBN), 1,5-Diazabicyclo-[5.4.0]undecen-5 (DBU) und Lutidin verwendet werden.

Die Arylmethylketone der allgemeinen Formel I werden in einer Gewichtsmenge zwischen 1 bis 40%, vorzugsweise zwischen 10 und 30%, bezogen auf die zu epoxidierende Verbindung, eingesetzt.

Die Reaktion wird bei einer Temperatur zwischen -5 und +60°C, vorzugsweise zwischen +10 und +40°C, durchgeführt.

Die Erfindung soll anhand der nachfolgenden Beispiele erläutert werden.

**BEISPIEL 1**

Zu einer Lösung von 5 g 3.3-(2.2-Dimethyltrimethylendioxy)-5(10),9(11)-estradien-17-on in 27 ml Dichlormethan gibt man 0.2 ml Pyridin, 1 g Trifluoracetophenon und 11,1 ml 30%iges $H_2O_2$. Anschließend erwärmt man 16 Stunden auf 40°C. Zur Aufarbeitung wäscht man mit Wasser, Natriumthiosulfatlösung und Wasser, trocknet und engt im Vakuum ein. Das erhaltene Rohprodukt wird an Aluminiumoxid mit Hexan/Essigester chromatographiert. Man erhält 425 mg 3.3-(2,2-Dimethyltrimethylendioxy-5β,10β-epoxy-9(11)-estren-17-on (8,1%) und 4,33 g 3.3-(2,2-Dimethyltrimethylendioxy)-5α, 10α-epoxy-9(11)-estren-17-on (83%).
Schmelzpunkt: 124,5-125,5°C (Hexan)

**BEISPIEL 2**

Zu einer Lösung von 5 g 3.3-(2.2-Dimethyltrimethylendioxy)-5(10),9(11)-estradien-17β-ol in 40 ml Dichlormethan gibt man 0,2 ml Pyridin, 1 g Trifluoracetophenon und 12 ml 30%iges $H_2O_2$. Anschließend erwärmt man 17 Stunden auf 40°C. Zur Aufarbeitung wäscht man mit Wasser, Natriumthiosulfatlösung und Wasser, trocknet und engt im Vakuum ein. Das erhaltene Rohprodukt wird an Aluminiumoxid mit Hexan/Essigester chromatographiert. Man erhält 565 mg 3.3-(2.2-Dimethyltrimethylendioxy)-5β, 10β-epoxy-9(11)-estren-17β-ol (10.8%) und 4.52 g 3.3-(2.2-Dimethyltrimethylendioxy)-5α, 10α-epoxy-9(11)-estren-17β-ol (86,6%).

**BEISPIEL 3**

5 g 3.3-(2.2-Dimethyltrimethylendioxy)-17β-hydroxy-17α-[3-(tetrahydropyran-2-yloxy) prop-1-inyl]-5(10),9(11)-estradien werden in 50 ml Dichlormethan gelöst und mit 0,25 ml Pyridin und 1,25 g p-Fluor-trifluoracetophenon und 13 ml 30%igem $H_2O_2$, 18 Stunden bei 40°C gerührt, mit Wasser, Natriumthiosulfatlösung und Wasser gewaschen, getrocknet und im Vakuum eingeengt. Das erhaltene Rohprodukt wird an Aluminiumoxid mit Hexan/Essigester chromatographiert. Man erhält 3,72 g 3.3-(2.2-Dimethyltrimethylendioxy)-5α, 10α-epoxy-17β-hydroxy-17α-[3-(tetrahydropyran-2-yloxy-prop-1-inyl]-9(11)-estren (72%).

**BEISPIEL 4**

Zu einer Lösung von 10 g 3.3-(2.2-Dimethyltrimethylendioxy)-5(10),9(11)-estradien-17-on in 60 ml Dichlormethan gibt man 0,5 ml 2.6-Lutidin und 2 g 2.4-Dichlor-trifluoracetophenon und 25 ml 30%iges $H_2O_2$. Anschließend rührt man 24 Stunden bei Raumtemperatur. Zur Aufarbeitung wäscht man mit Wasser, Natriumthiosulfatlösung und Wasser und engt im Vakuum ein. Das erhaltene Rohprodukt wird aus Hexan umkristallisiert. Man erhält 6,96 g 3.3-(2.2-Dimethyltrimethylendioxy)-5α, 10α-epoxy-9(11)-estren-17-on (68%).

**BEISPIEL 5**

Zu einer Lösung von 2 g 3.3-(2.2-Dimethyltrimethylendioxy)-5(10),9(11)-estradien-17-on in 30 ml Dichlormethan gibt man 0,2 ml Pyridin und 0,4 g 2.6-Dimethyl-trifluoracetophenon und 5 ml 30%iges $H_2O_2$. Anschließend rührt man 24 Stunden bei 40°C. Zur Aufarbeitung wäscht man mit Wasser, Natriumthiosulfatlö sung und Wasser und engt im Vakuum ein. Das erhaltene Rohprodukt wird an Aluminiumoxid mit Hexan/Essigester chromatographiert. Man erhält 1,27 g 3.3-(2.2-Dimethyltrimethylendioxy)-5α, 10α-epoxy-9(11)-estren-17-on (63%).

**BEISPIEL 6**

Unter den im Beispiel 1 beschriebenen Bedingungen erhält man, ausgehend von 5 g 3.3-(2.2-Dimethyltrimethylendioxy)-5(10),9(11)-estradien-17-on und 1,25 g 3-Methoxy-trifluoracetophenon nach Chromatographie, 3,96 g 3.3-(2.2-Dimethyltrimethylendioxy)-5α,10α-epoxy-9(11)-estren-17-on (78%).

**BEISPIEL 7**

Unter den im Beispiel 2 angegebenen Bedingungen, ausgehend von 5 g 3.3-(2.2-Dimethyltrimethylendioxy)-5(10),9(11)-estradien-17β-ol und 1,75 g Trichloracetophenon, erhält man nach Chromatographie 3,64 g 3.3-(2.2-Dimethyltrimethylendioxy)-5α,10α-epoxy-9(11)-estren-17β-ol (71%).

**BEISPIEL 8**

Zu einer Lösung von 1,0 g 3,3-(2,2-Dimethyltrimethylendioxy)-5(10),9(11)-estradien-17β-ol in 5 ml Dichlormethan gibt man 0,8 ml gesättigte Natriumhydrogencarbonatlösung, 1 ml 30%iges $H_2O_2$ und 245 mg 3-Nitrotrifluoracetophenon. Anschließend rührt man 3 Stunden bei Raumtemperatur. Zur Aufarbeitung wird nacheinander mit Wasser, Natriumthiosulfatlösung und Wasser gewaschen. Nach dem Trocknen der organischen Phase über Natriumsulfat wird filtriert, eingeengt und der Rückstand mit Hexan/Essigester an Aluminiumoxid chromatographiert. Man erhält so 145 mg (13,9 %) 3,3-(2,2-Dimethyltrimethylendioxy)-5β,10β-epoxy-9(11)-estren-17β-ol und 735 mg (70,3 %) 3,3-(2,2-Dimethyltrimethylendioxy)-5,10-epoxy-9(11)-estren17β-ol.

**Patentansprüche**

1. Verwendung eines Epoxidierungs-Reagenz bestehend aus Wasserstoffperoxid und mindestens einem Arylmethylketon der allgemeinen Formel I

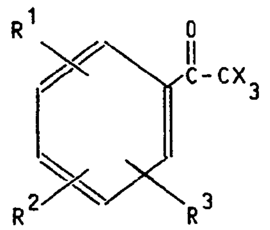

(I),

worin
X für ein Fluor- oder Chloratom,
$R^1$, $R^2$ und $R^3$, die gleich oder verschieden sein können, jeweils für einen $C_1$-$C_6$-Alkyl-, $C_6$-$C_{10}$-Aryl, $C_6$-$C_{10}$-Aryl-$C_1$-$C_6$-alkyl-, 0-$C_1$-$C_6$-Alkyl-, 0-$C_6$-$C_{10}$-Aryl-Rest, ein Fluor-, Chlor-, Brom- oder Wasserstoffatom, eine Cyano-, Nitro- oder Trifluormethylgruppe, einen Amidrest -CO-Nr$^4$R$^5$ oder einen Sulfonylrest -$SO_2R^4$, worin $R^4$ und $R^5$ gleich oder verschieden sind und jeweils eine $C_1$-$C_6$-Alkyl-, $C_1$-$C_{10}$-Aryl- oder eine $C_6$-$C_{10}$-Aryl-$C_1$-$C_6$-alkylgruppe, wobei im Falle von -CO-NR$^4$R$^5$ $R^4$ und $R^5$ gemeinsam einen aliphatischen oder aromatischen fünf- oder sechsgliedrigen Heterocyclus bilden können, bedeuten oder $R^1$ und $R^2$ gemeinsam für einen anellierten aliphatischen oder aromatischen Ring stehen, zur Herstellung von epoxidierten Steroiden.

2. Verfahren zur Herstellung von Steroiden mit einer 9(11)-Doppelbindung und einer 5α(10α)-Epoxigruppe, dadurch gerkennzeichnet, daß ein Steroid mit zwei Doppelbindungen in 5(10)- sowie in 9(11)-Stellung mit einem Epoxidierungs-Reagenz bestehend aus Wasserstoffperoxid und mindestens einem Arylmethylketon der allgemeinen Formel I

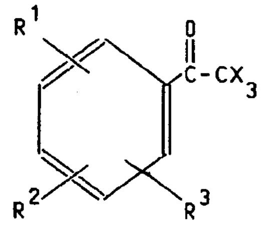

(I),

worin
X für ein Fluor- oder Chloratom,
$R^1$, $R^2$ und $R^3$, die gleich oder verschieden sein können, jeweils für einen $C_1$-$C_6$-Alkyl-, $C_6$-$C_{10}$-Aryl, $C_6$-$C_{10}$-Ar-$C_1$-$C_6$-alkyl-, 0-$C_1$-$C_6$-Alkyl-, 0-$C_6$-$C_{10}$-Aryl-Rest, ein Fluor-, Chlor-, Brom- oder Wasserstoffatom, eine Cyano-, Nitro- oder Trifluormethylgruppe, einen Amidrest -CO-NR$^4$R$^5$ oder einen Sulfonylrest -$SO_2R^4$, worin $R^4$ und $R^5$ gleich oder verschieden sind und jeweils eine $C_1$-$C_6$-Alkyl-, $C_1$-$C_{10}$-Aryl- oder eine $C_6$-$C_{10}$-Aryl-$C_1$-$C_6$-alkylgruppe, wobei im Falle von -CO-NR$^4$R$^5$ $R^4$ und $R^5$ gemeinsam einen aliphatischen oder aromatischen fünf- oder sechsgliedrigen Heterocyclus bilden können, bedeuten oder $R^1$ und $R^2$ gemeinsam für einen anellierten aliphatischen oder aromatischen Ring stehen, weitgehend stereospezifisch epoxidiert wird.

3. Verfahren nach Anspruch 5, dadurch gekennzeichnet, daß man bei einer Temperatur zwischen -5 bis +60°C, vorzugsweise zwischen +10 und +40°C, epoxidiert

4. Verfahren nach Anspruch 5 oder 6, dadurch gekennzeichnet, daß man die Epoxidierung in organischen Lösungsmitteln wie Dioxan, Diglyme, Tetrahydrofuran, Dimethoxyethan, oder in chlorierten organischen Lösungsmitteln unter Zusatz eines tertiären Amins, vorzugsweise in Dichlormethan, Chloroform oder Ethylenchlorid unter Zusatz von Pyridin oder Lutidin durchführt.

## Claims

1. The use of an epoxidation reagent comprising hydrogen peroxide and at least one aryl methyl ketone of the general formula I

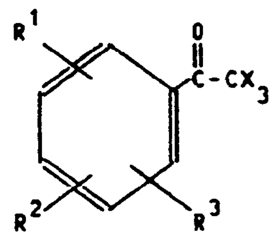

(I)

wherein
X represents a fluorine or chlorine atom,
$R^1$ , $R^2$ and $R^3$, which may be the same or different, each represents a $C_1$-$C_6$-alkyl, $C_6$-$C_{10}$-aryl, $C_6$-$C_{10}$-Ar-$C_1$-$C_6$-alkyl, 0-$C_1$-$C_6$-alkyl or 0-$C_6$-$C_{10}$-aryl radical, a fluorine, chlorine, bromine or hydrogen atom, a cyano, nitro or trifluoromethyl group, an amide radical -CO-$NR^4R^5$ or a sulphonyl radical -$SO_2R^4$, wherein $R^4$ and $R^5$ are the same or different and each represents a $C_1$-$C_6$-alkyl, $C_1$-$C_{10}$-aryl or $C_6$-$C_{10}$-aryl-$C_1$-$C_6$-alkyl group, it being possible in the case of -CO-$NR^4R^5$ for $R^4$ and $R^5$ together to form an aliphatic or aromatic five- or six-membered heterocycle, or $R^1$ and $R^2$ together represent a fused aliphatic or aromatic ring, for the preparation of epoxidised steroids.

2. Process for the preparation of steroids having a 9(11)-double bond and a 5α(10α)-epoxy group, characterised in that a steroid having two double bonds in the 5(10)- and 9(11)-positions is substantially stereo-specifically epoxidised with an epoxidation reagent comprising hydrogen peroxide and at least one aryl methyl ketone of the general formula I

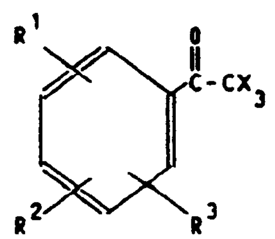

(I)

wherein
X represents a fluorine or chlorine atom,
$R^1$ , $R^2$ and $R^3$, which may be the same or different, each represents a $C_1$-$C_6$-alkyl, $C_6$-$C_{10}$-aryl, $C_6$-$C_{10}$-Ar-$C_1$-$C_6$-alkyl, 0-$C_1$-$C_6$-alkyl or 0-$C_6$-$C_{10}$-aryl radical, a fluorine, chlorine, bromine or hydrogen atom, a cyano, nitro or trifluoromethyl group, an amide radical -CO-$NR^4R^5$ or a sulphonyl radical -$SO_2R^4$, wherein $R^4$ and $R^5$ are the same or different and each represents a $C_1$-$C_6$-alkyl, $C_1$-$C_{10}$-aryl or $C_6$-$C_{10}$-aryl-$C_1$-$C_6$-alkyl group, it being possible in the case of -CO-$NR^4R^5$ for $R^4$ and $R^5$ together to form an aliphatic or aromatic five- or six-membered heterocycle, or $R^1$ and $R^2$ together represent a fused aliphatic or aromatic ring.

3. Process according to claim 2, characterised in that the epoxidation is carried out at a temperature of from -5 to +60°C, preferably from +10 to +40°C.

4. Process according to claim 2 or 3, characterised in that the epoxidation is carried out in organic solvents such as dioxan, diglyme, tetrahydrofuran, dimethoxy-ethane, or in chlorinated organic solvents with the addition

of a tertiary amine, preferably in dichloromethane, chloroform or ethylene chloride with the addition of pyridine or lutidine.

## Revendications

1. Utilisation d'un réactif d'époxydation constitué de peroxyde d'hydrogène et d'au moins une arylméthylcétone de la formule générale I

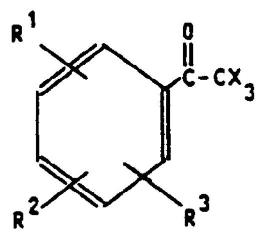

(I),

dans laquelle
X représente un atome de fluor ou de chlore,
$R^1$ , $R^2$ et $R^3$, qui peuvent être identiques ou différents, représentent chacun un radical C $_1$-$C_6$-alkyle, $C_6$-$C_{10}$-aryle, $C_6$-$C_{10}$-ar-$C_1$-$C_6$-alkyle, 0-$C_1$-$C_6$-alkyle, 0-$C_6$-$C_{10}$-aryle, un atome de fluor, de chlore, de brome ou d'hydrogène, un groupe cyano, nitro ou trifluorométhyle, un radical amide -CO-$NR^4R^5$ ou un radical sulfonyle -$SO_2R^4$, où $R^4$ et $R^5$ sont identiques ou différents et représentent chacun un groupe $C_1$-$C_6$-alkyle, $C_1$-$C_{10}$-aryle ou $C_6$-$C_{10}$-aryl-$C_1$-$C_6$-alkyle, $R^4$ et $R^5$ pouvant, dans le cas de -CO-$NR^4R$ , former conjointement un hétérocycle pentagonal ou hexagonal, aliphatique ou aromatique, ou $R^1$ et $R^2$ forment ensemble un noyau aliphatique ou aromatique condensé, pour la préparation de stéroïdes époxydés.

2. Procédé de préparation de stéroïdes présentant une double liaison 9(11) et un groupe époxy 5 $\alpha$(10$\alpha$), caractérisé en ce qu'on époxyde de manière largement stéréospécifique un stéroïde ayant deux doubles liaisons en position 5(10) ainsi que 9(11) par un réactif d'époxydation constitué de peroxyde d'hydrogène et d'au moins une arylméthylcétone de la formule générale I

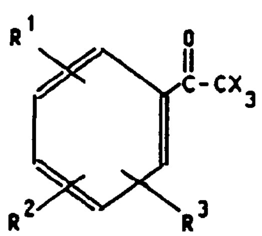

(I),

dans laquelle
X représente un atome de fluor ou de chlore,
$R^1$ , $R^2$ et $R^3$, qui peuvent être identiques ou différents, représentent chacun un radical $C_1$-$C_6$-alkyle, $C_6$-$C_{10}$-aryle, $C_6$-$C_{10}$-ar-$C_1$ -$C_6$-alyle, 0-$C_1$-$C_6$-alkyle, 0-$C_6$-$C_{10}$-aryle, un atome de fluor, de chlore, de brome ou d'hydrogène, un groupe cyano, nitro ou trifluorométhyle, un radical amide -CO-$NR^4R^5$ ou un radical sulfonyle -$SO_2R^4$, où $R^4$ et $R^5$ sont identiques ou différents et représentent chacun un groupe $C_1$-$C_6$-alkyle, $C_1$-$C_{10}$-aryle ou $C_6$-$C_{10}$-aryl-$C_1$-$C_6$-alkyle, $R^4$ et $R^5$ pouvant, dans le cas de -CO-$NR^4R^5$, former conjointement un hétérocycle pentagonal ou hexagonal, aliphatique ou aromatique, ou $R^1$ et $R^2$ forment conjointement un noyau aliphatique ou aromatique condensé.

3. Procédé suivant la revendication 2, caractérisé en ce qu'on époxyde à une température comprise entre -5 et +60°C, de préférence entre +10 et +40°C.

4. Procédé suivant l'une des revendications 2 et 3, caractérisé en ce qu'on effectue l'époxydation dans des solvants organiques, tels que du dioxane, des diglymes, du tétrahydrofuranne, du diméthoxyéthane, ou dans des solvants organiques chlorés, avec addition d'une amine tertiaire, de préférence dans du dichlorométhane, du chloroforme ou du chlorure d'éthylène, avec addition de pyridine ou de lutidine.